# EUROPEAN PATENT SPECIFICATION

(11) **EP 4 151 647 B1**
(45) Date of publication and mention of the grant of the patent: **31.12.2025**
(21) Application number: 22173183.9
(22) Date of filing: 13.05.2022
(51) Int. Cl.: C07K 14/005

(54) **AMINO ACID SEQUENCE OF RECOMBINANT TICK-BORNE ENCEPHALITIS VIRUS-LIKE PARTICLES AND THEIR USE AS A VACCINE ANTIGEN FOR PREVENTION OF TICK-BORNE ENCEPHALITIS VIRUS INFECTIONS**
AMINOSÄURESEQUENZ VON REKOMBINANTEN ZECKENENZEPHALITIS-VIRUS-ÄHNLICHEN PARTIKELN UND IHRE VERWENDUNG ALS IMPFSTOFFANTIGEN ZUR PRÄVENTION VON ZECKENENZEPHALITIS-VIRUS-INFEKTIONEN
SÉQUENCE D'ACIDES AMINÉS DE PARTICULES RECOMBINANTES RESSEMBLANT AU VIRUS DE L'ENCÉPHALITE À TIQUES ET LEUR UTILISATION COMME ANTIGÈNE VACCINAL POUR LA PRÉVENTION DES INFECTIONS PAR LE VIRUS DE L'ENCÉPHALITE À TIQUES

(30) Priority: 17.09.2021 PL 43896621
(43) Date of publication of application: 22.03.2023
(73) Proprietor: University of Gdansk, 80-309 Gdansk (PL)
(72) Inventor: Krol, Ewelina, 81-153 Gdynia (PL); Szewczyk, Boguslaw, 80-156 Gdansk (PL); Zimna, Marta, 80-627 Gdansk (PL); Brzuska, Gabriela, 66-105 Pomorsko (PL); Boch-Kminikowska, Sara, 80-824 Gdansk (PL)
(74) Representative: Czarnik, Maciej

(56) References cited:
- WO-A1-2013/116770
- WEIDMANN MANFRED ET AL: "Molecular phylogeography of tick-borne encephalitis virus in central Europe", JOURNAL OF GENERAL VIROLOGY, vol. 94, no. 9, 1 September 2013 (2013-09-01), pages 2129 - 2139, XP055980403, ISSN: 0022-1317, DOI: 10.1099/vir.0.054478-0
- DATABASE Geneseq [online] 25 March 2003 (2003-03-25), "Plasmid construct SV-PEwt protein.", XP002807969, retrieved from EBI accession no. GSP:AAW77407 Database accession no. AAW77407
- DE OLIVEIRA TATIANA APARECIDA ET AL: "Application of the LEXSY Leishmania tarentolae system as a recombinant protein expression platform: A review", PROCESS BIOCHEMISTRY, ELSEVIER LTD, GB, vol. 87, 27 August 2019 (2019-08-27), pages 164 - 173, XP085940819, ISSN: 1359-5113, [retrieved on 20190827], DOI: 10.1016/J.PROCBIO.2019.08.019
- BOLHASSANI AZAM ET AL: "Comparison of HCV Core and CoreE1E2 Virus-Like Particles Generated by Stably Transfected Leishmania tarentolae for the Stimulation of Th1 Immune Responses in Mice", CURRENT DRUG DELIVERY, vol. 14, no. 7, 16 October 2017 (2017-10-16), NL, XP093277344, ISSN: 1567-2018, DOI: 10.2174/1567201814666170125120917
- SCHALICH J ET AL: "Recombinant subviral particles from tick-borne encephalitis virus are fusogenic and provide a model system for studying flavivirus envelope glycoprotein functions", JOURNAL OF VIROLOGY, vol. 70, no. 7, 1 July 1996 (1996-07-01), US, pages 4549 - 4557, XP093277356, ISSN: 0022-538X, DOI: 10.1128/jvi.70.7.4549-4557.1996

## Description

The invention is related to the field of vaccines based on immunogenic antigens for the prevention of tick-borne encephalitis virus (TBEV) infections. The invention defines the amino acid sequence of recombinant virus-like particles (VLPs) containing two selected fragments of TBEV virus proteins and other inserted sequences. Two natural proteins of the TBEV virus - structural protein E and structural protein prM were used in the sequence. The invention in the form of the amino acid sequence of the recombinant virus-like particles has therapeutic utility in the prevention of TBEV infections as an immunogenic component for the preparation of vaccines. The subject of the invention is also medical use, in particular as an immunogenic vaccine against TBEV virus based on VLPs with the amino acid sequence according to the invention.

Viruses belonging to the genus *Flavivirus* of the *Flaviviridae* family include a large number of human pathogens as well as animal pathogens. Most of them are transmitted by insect vectors such as ticks, e.g., tick-borne encephalitis virus, and mosquitoes, e.g., Zika virus or Dengue virus.

TBEV is a pathogen that causes an infectious disease called tick-borne encephalitis (TBE). The main route of TBEV transmission to humans and animals are the bite of an infected tick, mainly of *Ixodes ricinus* or *Ixodes persulcatus* species, however other routes such as consumption of unpasteurized milk or dairy products from infected animals become increasingly important. It is believed that up to 20% of infections may occur in this way. There are three main subtypes of TBEV virus, previously separated geographically: European (TBEV-Eu), Siberian (TBEV-Sib) and Far Eastern (TBEV-FE) (Ruzek et al. (1999), Antiviral Res., 164: 23-51) and two newly discovered: Baikalian (TBEV-Bkl) and Himalayan (TBEV-Him).

The incubation time of the virus from infection to the disease development is usually from 7 to 14 days, however in some cases it may be even up to 28 days. Due to the possible asymptomatic course of infection, a large number of TBEV cases remain undiagnosed. In about 80% of cases that are correctly diagnosed, the infection is asymptomatic. Remaining 20% develops a disease, the initial course of which is similar to influenza - muscle and joint pain, fever, headaches and general fatigue. Several days after these symptoms appear, the virus can attack the nervous system, causing meningitis, encephalitis, or spinal cord inflammation. When a neurological infection is developed, the mortality rate depends on the virus subtype. In the case of the European subtype, the mortality rate ranges from 1 to 5%, for the Siberian subtype 2-8%, and in the case of the Far Eastern subtype, it ranges from 20 to 60% (Ecker et al. (1999), J. Gen. Virol., 80 (1): 179-185).

The TBEV virus was isolated for the first time in 1937 in Soviet Union. So far, its presence has been demonstrated in 28 countries in Central and Eastern Europe, Scandinavia and selected regions of Asia. Every year there are new outbreaks of infection in many countries, e.g. in Finland, Denmark, Norway, Austria, Germany and Poland (Yoshii et al. (2019), J. Vet. Med. Sci., 81 (3): 343-347). The incidence of TBEV infections has increased more than 4 times in the last 20 years, which makes tick-borne encephalitis the second most common disease transmitted by ticks after Lyme disease (Daep et al. (2014), J Neurovirol., 20 (6): 539- 60).

Flaviviruses are characterized by high structural similarity of virions, similarities in the course of the replication cycle, as well as high similarity of individual viral proteins. TBEV virus, like other flaviviruses, is composed of an icosahedral capsid formed by the structural protein C. In the center of the capsid there is the viral genome in the form of ssRNA (+). From the outside, the capsid is covered with a lipid bilayer in which two structural proteins are anchored: prM / M and glycoprotein E. Mature virions are composed of 90 dimers of glycoprotein E and 90 dimers of mature protein M. Both prM / M and E proteins are transmembrane proteins with two transmembrane domains at the C 'terminus. The structure of glycoprotein E can be divided into three domains (DI, DII, DIII), a region of helical domains (stem) and a region of transmembrane domains (anchor). DI is β-barrel shaped, DII is similar in the structure to the zinc finger domain, while DIII is structurally related to immunoglobulin. Most flavivirus strains possess a single N-linked glycosylation site in DI. According to functional studies concerning the structure of TBEV glycoprotein E, the role of DII in the dimerization process has been demonstrated, while the stem region was shown to participate in the trimerization and stabilization of the prM-E complex (Füzik et al. (2018), Nat Commun. 30; 9 (1) : 436). The anchor region is likely involved in viral particle assembly. There are many epitopes in DIII that are recognized by neutralizing antibodies and cytotoxic lymphocytes, which makes glycoprotein E a suitable candidate for use as a vaccine or diagnostic antigen. During the TBEV replication cycle, virion assembly begins in the endoplasmic reticulum, where the newly formed polyprotein containing all the viral proteins is proteolytically processed. In the first step, protein C is cleaved by the NS2B / NS3 viral protease, then the prM and E proteins are cleaved by the signal peptidase I of the host cell. At this stage, spherical viral particles are formed in an immature form, composed of prM and E proteins anchored in the lipid envelope. The immature particles are then transported to the Golgi apparatus and further to the secretory vesicles of the trans Golgi apparatus. The pr fragment of the prM protein covers the E protein fusion loop to prevent premature fusion of the envelope with the membrane of the secretory vesicles caused by their acidic environment. Just before the virions are released from the host cell, the pr fragment is cleaved from the prM protein by the furin protease present in the trans Golgi network, which leads to the secretion of mature viral particles (Pulkkinen et al. (2018), Viruses, 10 (7): 1-20). However, the cleavage by this protease is ineffective, resulting in the release of a mixed population of both mature and immature virions from the cell. It is believed that the presence of immature flavivirus virions in the human body may play a role in avoiding an immune response. Moreover, during assembly of virions, spherical particles structurally resembling viral particles are also formed, composed of only two structural proteins, prM / M and E, anchored in the cell membrane. These particles, however, are lacking both capsid and a genetic material. The natural process of particle assembling and flaviviral virions has been used to produce recombinant virus-like particles in various gene expression systems. Gene expression of the two structural proteins prM and E leads to the formation of VLPs. VLPs consist of multiple copies of the prM / M and E proteins anchored to the lipid bilayer. The high structural similarity to native virions makes VLPs an excellent candidate as vaccine antigens to replace live attenuated or inactivated viruses. The lack of genetic material makes them unable to replicate, and at the same time they are highly immunogenic, as they contain many copies of viral proteins, which enables the appropriate presentation of epitopes that are the target for neutralizing antibodies or cytotoxic lymphocytes.

Five anti-TBEV virus vaccines based on inactivated virus are available currently on the market. Two of them are based on the strains of European subtype - FSME-IMMUN (Pfizer / USA) based on Neudoerfl strain and Encepur (GlaxoSmithKline plc / UK) on the K23 strain. Another vaccines based on Far Eastern strains are approved in Russia: TBE vaccine Moscow and Tick-E-Vac (Chumakov FSC R&D IBP RAS / Russia) both based one Sofjin strain and EnceVir (Microgen-Branch FSUC "SIC" Microgen "of MOH of Russia "SIC" Virion "/ Russia) based on strain 205. All these vaccines are produced in primary chicken embryo fibroblasts cells (PCECs). After antigen purification and inactivation, the adjuvants and stabilizers are added to formulate a vaccine. All vaccines are adjuvanted with aluminum hydroxide. Sucrose or human serum albumin are used as stabilizers. The SenTaiBao (Changchun Institute of Biological Products Co., Ltd./China) vaccine based on the Sen-Zhang (Far East) strain has also been developed but it has only been approved in China and has not been used for at least 9 years. All described vaccines are multi-dose; require two or three doses at specific intervals, followed by booster doses every 3-5 years. Depending on the formulation, the effectiveness of the available vaccines in different studies is reported as ranging from 86 to 99% (Kubinski et al. (2020), Viruses 12; 8 (3): 451).

In addition to the available vaccine formulations, research using other approaches in the development of anti-TBEV vaccines is underway. One of the first approaches was antigens based on recombinant protein E or VLPs (prM and E proteins). In mice studies, it was shown that the formation of higher protein structures and VLPs increased the level of the immune response (Heinz et al. (1995), Vaccine, 13: 1636-1642). Another approach was to use a DNA vaccine carrying genes for the prM-E structural proteins. In mice and primates studies the effectiveness was shown to be comparable to the inactivated FSME-IMMUN vaccine. It was also more effective than an analogous approach in which the DNA vaccine harbored only the E protein gene (Aberle et al. (1999), J. Immunol. 163: 6756-6761; Schmaljohn et al. (1997) J. Virol., 71: 9563-9569; Schmaljohn et al. (1999) Virology, 263: 166-174). RNA vaccines have also been shown to be effective. In one approach infectious RNA with an introduced mutation that allows the attenuation of the virus was used (Mandl et al. (1998), Nat. Med. 4: 1438-1440). In another study, the RNA covered the entire viral genome, however, due to the deletion of a fragment of C protein, preventing the assembly of progeny virions, it was non-infectious (Kofler et al. (2004), Proc. Natl. Acad. Sci., 101: 1951-1956; Aberle et al. (2005), J. Virol., 79: 15107-15113). In both cases, mice studies showed elicitation of a strong immune response, including a cellular response that was not elicited by the use of FSME-IMMUN. The vaccine preparations used in these studies contained 'naked' RNA with no additional carriers.

In addition to the approaches described above, the effectiveness of vector vaccines based on adenoviruses and vaccinia virus was also investigated. In most cases, the genes encoding the prM and E proteins or the NS1 nonstructural protein were inserted into the vector. In studies in mice, vector vaccines were shown to be highly effective. Many studies using attenuated virus vaccines have also been performed, but in most cases the level of viral attenuation was insufficient, or the effectiveness of the vaccine canditate was lower than when using other approaches (Kubinski et al. (2020), Viruses 12; 8 (3): 451).

Many strategies for the production of flaviviral recombinant proteins and VLPs that can be used as vaccine or diagnostic antigens have been applied and described in the literature. These strategies are based on the expression of genes for the structural proteins C, prM and / or E in a natural or modified - recombinant form. The presented invention refers to a new strategy for the production of immunogenic particles, i.e., recombinant virus-like particles. This strategy includes the combination of the natural amino acid sequences of the TBEV prM and E proteins with other additional elements, i.e., the natural signal sequence for secreted acid phosphatase 1 (LMSAP1) from *Leishmania mexicana* and the natural P2A peptide sequence together with the GSG linker being the glycine-serine-glycine amino acids combination. The introduced modifications improve the production, assembly, secretion, maturation and immunogenicity of obtained recombinant proteins and VLPs. Recombinant VLPs based on the amino acid sequence presented in this invention can be overproduced in an expression system in protozoan cells.

The first aspect ofthe present invention relates to the amino acid sequence of the recombinant virus-like particles (VLPs) shown as the sequence number SEQ 1, based on the natural TBEV proteins: the prM and E protein. The change in the sequence of natural proteins refers, inter alia, to the introduction of additional modified sequences into the sequence of natural proteins, i.e., the signal sequence for LMSAP1 phosphatase from *Leishmania mexicana,* e.g., the signal sequence from the LEXSinduce3 Expression Kit (Jena Bioscience) expression system and P2A sequence, which result in more efficient overproduction of VLPs and increase their immunogenicity.

A second aspect of the invention relates to recombinant VLPs with the amino acid sequence according to the invention (SEQ 1) for medical use in the prevention and control of tick-borne encephalitis, especially when used in a form of a vaccine component. This aspect includes the composition of an immunogenic preparation containing VLPs preferably together with an adjuvant. As an adjuvant, a nanoemulsion of squalene in water can be used, e.g., AddaVax (InvivoGen), 2% aluminum hydroxide gel suspension - Alhydrogel. The use of an adjuvant increases the immunogenicity of the preparared potential vaccine.
The invention is disclosed in the claims.
The first aspect of the invention, i.e., the amino acid sequence of the recombinant VLPs (SEQ 1) according to the invention, are the amino acid sequences of the TBEV structural proteins into which various sequence modifications have been made.

The basic structural pram and E proteins of TBEV virus are the natural components of the virions. In the TBEV genome, the prM and E protein genes follow the structural C protein gene. In the prior art, for the TBEV virus, the transmembrane domain of the protein C is a natural signal sequence for the prM protein, and similarly the transmembrane domain of the prM protein is the natural signal sequence for the E protein. In the present invention it was not used but instead a signal sequence for LMSAP1 phosphatase from *Leishmania mexicana* was introduced. In general, signal sequences direct proteins to appropriate compartments in a eukaryotic cell for their further maturation, assembly into viral particles, and secretion outside the cell. After fulfilling their role, these signal sequences are then cut off by signal peptidases appropriate for a eukaryotic cell type. The *Leishmania mexicana* LMSAP1 phosphatase signal sequence was used in the invention to improve the properties of recombinant VLPs. This sequence was introduced upstream of the prM protein, replacing the natural signal sequence for the protein (transmembrane domain of C protein). LMSAP1 phosphatase from *Leishmania mexicana* is a naturally secreted protein in protozoan cells. Its signal sequence is added to the VLPs sequence to increase the efficiency of overproduction and efficient maturation and secretion of recombinant VLPs in *Leishmania tarentolae* cells.

Another modification is the introduction of a natural P2A peptide sequence derived from the virus described below, responsible for the self-acting separation of the co-maturing recombinant prM and E proteins, which can form VLPs, eliminating the dependence of this process on the presence of the appropriate signal peptidase in the eukaryotic cell. Efficient separation of prM and E proteins is essential for the full maturation of virus-like particles. The P2A peptide sequence orginates from the porcine Teschovirus-1. It is a 19-amino acid peptide terminated with proline-glycine-proline amino acids. The presence of these amino acids in the protein cassette causes the termination of translation at the first proline site and to restart of translation from the next proline, resulting in the production of two separate recombinant proteins. Additionally, the "GSG linker" between the prM protein and the P2A peptide was introduced. This linker is a sequence of three amino acids of glycine-serine-glycine, between the C 'terminus of the prM protein and the N' terminus of the P2A peptide in order to reduce spherical disorders of the protein structure.

Based on the natural ability of prM and E proteins to form VLPs, on the previous studies with flaviviral VLPs as well as on our own experience in the production of viral particles performed at the Department of Recombinant Vaccines at the Intercollegiate Faculty of Biotechnology of the University of Gdańsk and Medical University of Gdańsk, a new and effective strategy for the production of flaviviral recombinant VLPs containing known TBEV structural proteins, using a combination of the above-mentioned elements.
The combinations of the presented modifications in the amino acid sequences of the known TBEV proteins, i.e., the amino acid sequences of the invention, are shown in the Scheme 1. The amino acid sequences of the TBEV prM and E proteins can be derived from different strains of TBEV. The amino acid sequences of the TBEV-Neudoerfl strain (GenBank: AAA86870.1) proteins were used in the recombinant proteins and VLPs presented in the invention. The invention is also illustrated in the sequence listing.

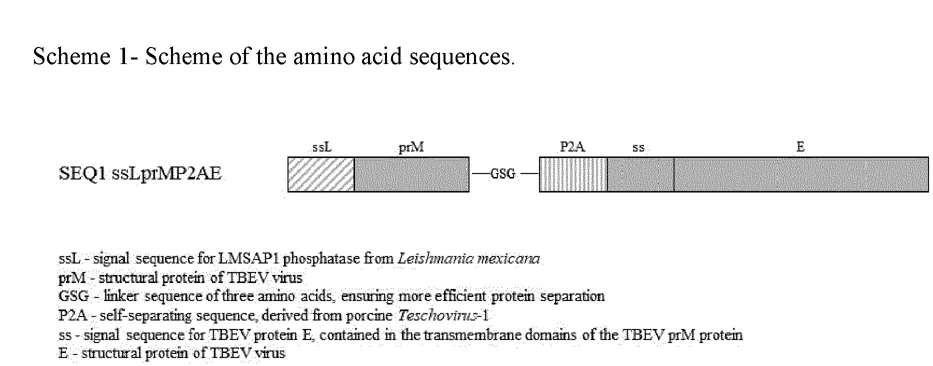
Scheme 1- Scheme of the amino acid sequences.

ssL prM P2A ss E
SEQ1 ssLprMP2AE —GSG—

ssL - signal sequence for LMSAP1 phosphatase from Leishmania mexicana
prM - structural protein of TBEV virus
GSG - linker sequence of three amino acids, ensuring more efficient protein separation
P2A - self-separating sequence, derived from porcine Teschovirus-1
ss - signal sequence for TBEV protein E, contained in the transmembrane domains of the TBEV prM protein
E - structural protein of TBEV virus

### SEQ 1

The amino acid sequence of recombinant VLPs - the amino acid sequence consisting of the amino acid sequences of two proteins: prM (region 114-281 in the amino acid sequence of the TBEV virus polyprotein) and E of the TBEV virus (region 282-776 in the amino acid sequence of the TBEV virus polyprotein) preceded by the amino acid signal sequence for LMSAP1 phosphatase from *Leishmania mexicana* (region 1-23 in the amino acid sequence of this protein, Genbank: CAA87090.1) e.g. signal sequence from the LEXSinduce3 Expression Kit (Jena Bioscience) expression system, separated by a linker sequence of the three amino acids described above - glycine-serine-glycine referred to as GSG linker fused to P2A peptide (region 979-997 of the amino acid sequence of *Teschovirus*-1 polyprotein, Genbank: NP_653143.1).

In summary, the introduced modifications to the amino acid sequences of the known flaviviral proteins described in this invention offers the following advantages:
- the signal sequence for LMSAP1 phosphatase from *Leishmania mexicana* facilitates the secretion of mature virus-like VLPs (SEQ 1), which allows for the usage of the simpler methods of VLPs purification
- the P2A peptide inserted between the prM and E proteins (SEQ 1) allows for more efficient separation of the flaviviral prM and E proteins, which improves the efficiency of VLPs particles assembly- the signal sequence for the LMSAP1 phosphatase from *Leishmania mexicana* used as the signal sequence for the prM and P2A proteins inserted between the prM and E proteins (SEQ 1) increases the yield of recombinant TBEV VLPs in the *Leishmania tarentolae* expression system LEXSinduce3 Expression Kit (Jena Bioscience).

Virus-like particles (VLPs) based on SEQ 1 sequence can elicit a strong immune response in a mouse model, which is necessary for the effective neutralization of TBEV virus in both *in vitro* and *in vivo* models, which translates into the neutralization of TBEV virus in the human body.

The VLPs of SEQ 1 according to the invention may be overproduced in *Leishmania tarantolae* protozoan cells. For overproduction, the commercial LEXSY overproduction system (Jena Bioscience) can be used.

For the overproduction of flaviviral antigens, standard conditions (e.g., culture medium composition, overproduction time, cell culture temperature, amount of vector used, etc.) used for overproduction of viral proteins and / or conditions recommended by the commercial system manufacturers may be used.

The second aspect of this invention concerns the use of recombinant VLPs based on sequence SEQ 1 according to the invention as a vaccine antigen in the prophylaxis of TBEV infections. This aspect also includes a composition of an immunogenic formulation containing VLPs and preferably at least one adjuvant - a vaccine component. AddaVax (InvivoGen) or Alhydrogel (InvivoGen) can be used as an adjuvant.

A vaccine formulation to prevent TBEV infection may consist of produced recombinant VLPs of SEQ 1 mixed with an adjuvant. Recombinant VLPs can be produced according to the production method outlined in Example 1. The schedule of administration of such vaccine preparations may include 1 to 3 doses administered in 2 weeks intervals. One dose may contain 1-100 µg of total protein in the sample suspended in 50-100 µl of PBS buffer mixed in a 1:1 ratio with an adjuvant.

Vaccine preparations can be administered intramuscularly and / or subcutaneously.
The invention is shown in the sequence described hereinafter as SEQ 1. The invention has also been described with the following working examples, figures and tables:
Figure 1. Western blot analysis showing the production of recombinant ssLprMP2AE VLPs (SEQ 1) in culture medium from *Leishmania tarantolae* protozoan cells.
Fig. 2. Transmission electron microscopy of VLPs ssLprMP2AE (SEQ 1) (negative staining and negative staining with Immunogold labeling with anti-protein E antibodies).
Figure 3. The graph showing mean titers of mouse sera obtained after 3 immunizations with VLPs with ssLprMP2AE sequence SEQ 1.
In addition, in Table 1. TBEV neuralization with of mouse sera obtained after 3 immunizations with VLPs with ssLprMP2AE sequence SEQ 1 was included.

### Example 1

### PRODUCTION OF RECOMBINATED VLPs WITH AMINO ACID SEQUENCE ACCORDING TO THE INVENTION

The sequence was obtained in a known procedure.

The genes encoding the recombinant VLPs ssLprMP2AE with the sequence SEQ 1 were chemically synthesized by GeneArt (Life Technology). They were then cloned into the pLEXSY_I-blecherry3 plasmid vector - a component of the LEXSinduce3 Expression Kit (Jena Bioscience) expression system using SalI / NotI restriction sites.

The plasmid was propagated in *E. coli* cells (TOP10) and isolated using a Plasmid Mini kit (A&A Biotechnology). The plasmid was then lined up with the restriction enzyme SwaI for the introduction into *Leishmania tarentolae* cells. The plasmid prepared in this way was used for transfection according to the protocol of the LEXSinduce3 Expression Kit system (Jena Bioscience) in order to obtain inducible stable lines carrying the genes encoding the VLPs. After transfection by electroporation, cells were selected with bleomycin (100 µg / ml) according to the manufacturer's protocol. Cells were cultured in LEXSY BHI Medium (Jena Bioscience) supplemented with hemin (5 µg / ml), Pen-Strep and LEXSY NTC (100 µg / ml) (Jena Bioscience) and Hygromycin B Gold (100 µg / ml) (InvivoGen) at 26 ° C. After 2 weeks of selection, a stable line was obtained with inducible expression of genes encoding the recombinant proteins forming ssLprMP2AE VLPs under the control of the tetracycline repressor.

For overproduction of VLPs based on SEQ 1, cells were induced with tetracycline (15 µg / ml), 50 ml culture scale, prepared by inoculating 5 ml of the culture into 45 ml of fresh LEXSY BHI Medium (Jena Bioscience). The cells were then incubated for 3 days at 26 ° C with shaking.

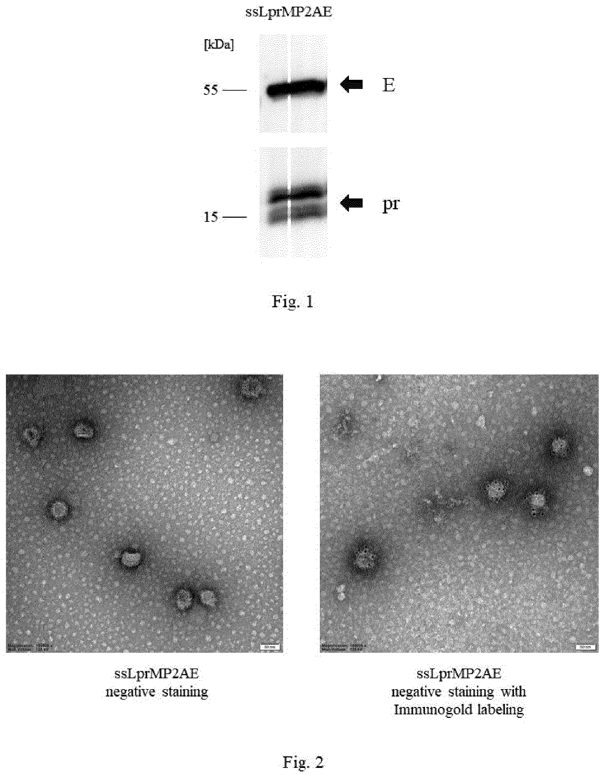

In a next step, the supernatant medium was centrifuged to get rid of cell debris (8000 rpm, 15 min). Western blot analysis was performed using anti-E and prM protein antibodies to confirm the presence of recombinant VLPs based on SEQ 1 in the culture medium. Fig. 1 shows the result of the analysis performed, confirming the production and secretion of the prM and E proteins into the medium. For VLPs purification, the medium was pelleted by ultracentrifugation through a 20% sucrose cushion (20% sucrose in Tris-NaCl-EDTA 50 mM-100 mM-10 mM buffer) at 28,000 rpm for 3h. The precipitates were dissolved overnight in phosphate buffered saline pH 7.4 (PBS). After dissolution, the precipitates were subjected to ultracentrifugation in a sucrose density gradient (20-60% in Tris-NaCl-EDTA 50 mM-100 mM-10 mM buffer), 27,000 rpm, 16 h. Amicon Ultra 100K filters (Merck Milipore) were used to remove sucrose from the samples and exchange the buffer with PBS.
Analysis of fractions containing VLPs ssLprMP2AE (SEQ 1) after gradient ultracentrifugation was performed using various methods such as western blotting, coomassie brilliant blue staining, transmission electron microscopy, Bradford method to determine protein concentration. The results of the analysis are shown in Fig. 2. Both negative staining electron microscopy and negative staining with Immunogold labeling with specific antibodies against protein E (Fig. 2) and the other mentioned test methods confirmed that the proteins produced according to SEQ 1 were assumed to form VLPs.

### Example 2

### VACCINE PREPARATION

The structure of the sequences according to the invention allows the medical use of VLPs for the prevention of TBEV infections from the *Flavivirus* genus, especially as a component of a vaccine.

For instance, recombinant VLPs based on the SEQ 1 sequence produced by the method presented in Example 1 were used as an immunogen in the mouse model study.
The vaccine preparations with the composition described above were administered to mice in 3 doses with an interval of 2 weeks. A single dose of vaccine was prepared by mixing the purified recombinant VLPs (SEQ 1) (10 µg total amount of purified protein in 100 µl volume) with AddaVax adjuvant (InvivoGen) in a 1:1 volume ratio or Alhydrogel adjuvant (InvivoGen) in a 1:25 mass ratio. The preparations were then administered to two groups of BALB/c mice (6 female mice per group) subcutaneously at two injection sites. The control group of three BALB/c mice was inoculated with a PBS buffer/adjuvant mixture in the same ratio.

After the first inoculation, the next inoculations (2 and 3) were made by mixing 10 µg of the purified recombinant VLPs (SEQ 1) in a volume of 50 µl mixed in a 1: 1 volume ratio with AddaVax adjuvant or 1:25 weight ration with Alhydrogel adjuvant. The preparations were also administered subcutaneously at one injection site.

In order to evaluate the efficacy of potential vaccines, i.e., the immunogenicity of recombinant VLPs, sera were collected after each vaccination from each group of mice and the immunogenicity analysis was performed by determining the serum titer. The titre was determined by ELISA test using prM / E proteins overproduced in mammalian HEK293T cells and purified by ultracentrifugation or on a nickel matrix using histidine tags as antigens. Briefly, M-96 plate was coated overnight with 10 µg / ml of antigen at 50 µl. A blocking solution (3% BSA in PBS + 0.05% Tween20) was then added (100 µl). After washing, serial dilutions of sera obtained from individual mice in a volume of 50 µl were added and incubated for 2 h. Then, after washing, secondary anti-mouse antibodies conjugated to horseradish peroxidase were added and incubated for 1 h. After washing, the substrate tetramethylbenzidine was added (TMB) (Life Technology) and an enzymatic reaction was performed for 2 min. The reaction was then stopped by adding 0.5 M sulfuric acid (VI). The reaction was read by reading the absorbance at 450 nm in a plate reader.

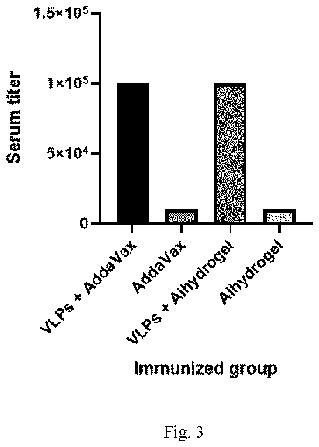

The serum titer was determined by selecting a serum dilution in which the signal is three times higher than the signal in the appropriate dilution of sera from control mice. The serum titers are presented in Figure 3. After the third vaccination, the average serum titers were about 10⁵, which indicates the high immunogenicity of the vaccine preparation used containing recombinant VLPs.

The neutralizing potency of the sera from immunized mice was determined in order to further evaluate the effectivity of the immunization with VLPs. Sera were inactivated by heat (56°C for 30 min) and diluted 1:4 in Leibowitz L-15 medium supplemented with 3% fetal bovine serum, 100 U/mL penicillin, 100 µg/mL streptomycin, and 1% glutamine. Subsequently, serial dilutions of the samples in L-15 medium (50 µL/well) were incubated with 10³ PFU/mL of TBEV strain Hypr (50 µL/well) in 96-well plates for 90 minutes at 37°C. The virus dose was adjusted to produce a near confluent cytopathic effect with 90-95% cytolysis. Porcine kidney stable cells (PS) were then added (3×10⁴ cells in 100 µL per well). After 5 days of incubation, the cytopathic effect was examined using an inverted microscope. The highest serum dilution that inhibited the cytopathic effect of the virus was considered the endpoint titer. Samples with a titer of 1:20 and higher were considered positive for the presence of anti-TBEV neutralization antibodies.

The mean dilutions in which sera of immunized mice neutralized TBEV were 1: 160 for mice vaccinated with VLPs in combination with AddaVax adjuvant and 1: 115 for mice vaccinated with VLPs and Alhydrogel. The obtained results confirm the immunogenicity of the obtained VLPs based on SEQ 1 according to the invention and confirm their usefulness as a vaccine preparation. The results are shown in Table 1.

**Table. 1**

| **group** | **serum dilution** |
|---|---|
| VLPs+AddaVax | 1:160 |
| PBS+AddaVax | lack of neutralization |
| VLPs+Alhydrogel | 1:115 |
| PBS+Alhydrogel | lack of neutralization |

## Claims

1. A recombinant virus-like particles of tick-borne encephalitis virus comprising a polypeptide of SEQ ID NO: 1, wherein SEQ ID NO: 1 comprises amino acid sequences of prM protein region 114-281 in the amino acid sequence of the tick-borne encephalitis virus (TBEV) polyprotein, amino acid sequences of E protein of the TBEV region 282-776 in the amino acid sequence of the TBEV virus polyprotein and amino acid signal sequence for LMSAP1 phosphatase from *Leishmania mexicana* region 1-23 in the amino acid sequence of this protein, wherein the signal sequence of LMSAP1 is situated before the sequence of prM protein and in addition the final sequence comprises linker sequence of the three amino acids being glycine-serine-glycine linked with peptide P2A region 979-997 in amino acid sequence of Teschovirus-1 polyprotein, wherein the recombinant virus-like particles are designed for production in an expression system based on *Leishmania tarentolae.*

2. The recombinant virus-like particles according to claim 1 for use in the prevention and treatment of tick-borne encephalitis.

3. A composition comprising the recombinant virus-like particles according to claim 1 and an adjuvant for use as a vaccine against tick-borne encephalitis.

## Patentansprüche

1. Rekombinante virusähnliche Partikel des durch Zecken übertragenen Enzephalitisvirus (TBEV), umfassend ein Polypeptid von SEQ ID NO: 1, wobei SEQ ID NO: 1 Aminosäuresequenzen des prM-Proteins der Regionen 114-281 in der Aminosäuresequenz des TBEV-Polyproteins, Aminosäuresequenzen des E-Proteins der TBEV-Region 282-776 in der Aminosäuresequenz des TBEV-Polyproteins sowie eine Aminosäure-Signalsequenz der LMSAP1-Phosphatase aus *Leishmania mexicana* der Region 1-23 in der Aminosäuresequenz dieses Proteins umfasst, wobei die Signalsequenz von LMSAP1 vor der Sequenz des prM-Proteins liegt und zusätzlich die Endsequenz eine Verbindungssequenz aus drei Aminosäuren (Glycin-Serin-Glycin) umfasst, die mit dem Peptid P2A der Region 979-997 der Aminosäuresequenz des Teschovirus-1-Polyproteins verknüpft ist, wobei die rekombinanten virusähnlichen Partikel für die Produktion in einem auf *Leishmania tarentolae* basierenden Expressionssystem ausgelegt sind.

2. Die rekombinanten virusähnlichen Partikel gemäß Anspruch 1 zur Verwendung in der Prävention und Behandlung der durch Zecken übertragenen Enzephalitis.

3. Eine Zusammensetzung umfassend die rekombinanten virusähnlichen Partikel gemäß Anspruch 1 und ein Adjuvans zur Verwendung als Impfstoff gegen die durch Zecken übertragene Enzephalitis.

## Revendications

1. Particules recombinantes de type viral du virus de l'encéphalite à tiques comprenant un polypeptide de la SEQ ID NO: 1, où la SEQ ID NO: 1 comprend les séquences d'acides aminés de la protéine prM des régions 114-281 dans la séquence d'acides aminés du polyprotéine du virus TBEV, les séquences d'acides aminés de la protéine E de TBEV des régions 282-776 dans la séquence d'acides aminés du polyprotéine du TBEV, ainsi que la séquence signal de la phosphatase LMSAP1 de *Leishmania mexicana* (région 1-23) dans la séquence d'acides aminés de cette protéine ; la séquence signal de LMSAP1 étant située avant la séquence de la protéine prM, et la séquence finale comprenant en outre une séquence de liaison de trois acides aminés - glycine-sérine-glycine - reliée au peptide P2A de la région 979-997 dans la séquence d'acides aminés du polyprotéine de Teschovirus-1, lesdites particules de type viral recombinantes étant conçues pour être produites dans un système d'expression basé sur *Leishmania tarentolae.*

2. Les particules recombinantes de type viral selon la revendication 1 pour une utilisation dans la prévention et le traitement de l'encéphalite à tiques.

3. Une composition comprenant les particules recombinantes de type viral selon la revendication 1 et un adjuvant, destinée à être utilisée comme vaccin contre l'encéphalite à tiques.
